(19) Europäisches Patentamt
European Patent Office
Office européen des brevets

(11) Publication number: **0 582 469 A2**

(12) # EUROPEAN PATENT APPLICATION

(21) Application number: **93306158.2**

(22) Date of filing: **04.08.93**

(51) Int. Cl.$^5$: **C07D 305/14,** C07C 233/87, C07D 205/08

(30) Priority: **06.08.92 US 926738**

(43) Date of publication of application: **09.02.94 Bulletin 94/06**

(84) Designated Contracting States: **BE CH DE DK ES FR GB IE IT LI NL PT**

(71) Applicant: **WISCONSIN ALUMNI RESEARCH FOUNDATION**
**P.O. Box 7365**
**Madison, WI 53707-7365 (US)**

(72) Inventor: **Sih, Charles J.**
**6322 Landfall Drive**
**Madison, Wisconsin 53705 (US)**

(74) Representative: **West, Alan Harry et al**
**R.G.C. Jenkins & Co. 26 Caxton Street**
**London SW1H 0RJ (GB)**

(54) **Process for preparing taxol-type compound from beta-lactam precursors.**

(57) Taxol-type compounds are produced by coupling baccatin III-type compounds (and related alcohols) with chemoenzymatically produced β-lactam enantiomers. The β-lactam enantiomers are produced from racemic starting substituted β-lactams through lipase contacting to achieve either enantioselective hydrolysis or enantioselective transesterification (the latter with an added 1-substituted ethenyl acetate). Preferred β-lactam enantiomers are coupled to the C-13 hydroxyl of the starting baccatin III-type compounds which are derived from 10-deacetyl baccatin III. Certain of the β-lactam enantiomers are new as are taxol-type compounds produced by the coupling reaction.

EP 0 582 469 A2

Jouve, 18, rue Saint-Denis, 75001 PARIS

This invention relates to a process for preparing taxol-type compounds from 10-deacetyl baccatin III-type compounds and substituted lactams. The invention further relates to the chemoenzymatic synthesis of these β-lactams.

Taxol is a naturally occurring diterpene which demonstrates excellent activity against melanomas and mammary and ovarian carcinomas. A significant disadvantage to the isolation of the natural occurring product is that the bark of five to seven 200 year old Pacific yew trees is needed to produce enough taxol to treat one patient with ovarian cancer. A practical synthetic route for producing taxol is needed.

Of the various synthetic routes that have been proposed, those which are based on the addition to the C-13 hydroxyl group of 10-deacetyl baccatin III of the β-amido ester group-containing side chain (also identifiable as the 3-phenylisoserine moiety) are promising. 10-Deacetyl baccatin III has the structure:

(1)

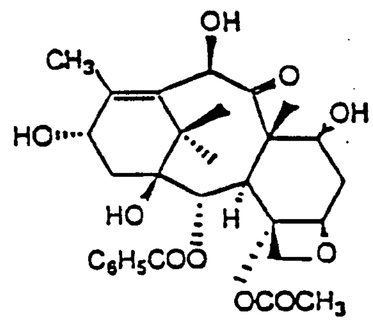

and is obtainable in practical quantities and at practical cost from the (renewable) needles of the abundant ornamental bush Taxus baccata and has been shown to be convertible into taxol which has the structure:

(2)

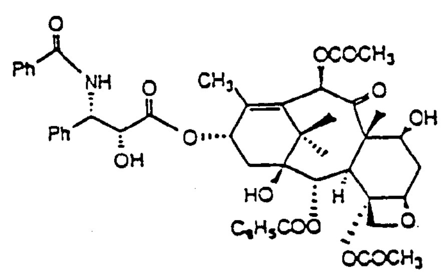

by reaction with either (a) a derivative of N-benzoyl-(2R,3S)-3-phenyl isoserine [Greene et al., J. Am. Chem. Soc., 110, 5917-5919 (1988)], (b) a derivative of a substituted oxazinone [R. A. Holton, U.S. Pat. No. 5,015,744] or (c) a derivative of a 3-hydroxy-4-aryl-β- lactam [R. A. Holton EPA 0 400 971 A2]. The taxol C-13 side chain, i.e., the N-benzoyl-(2R,3S)-3-phenyl isoserine enantiomeric moiety, has the structure:

(3)

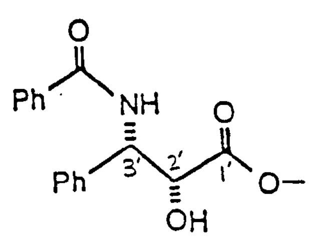

and has been shown to be essential for the observed potent antitumor activity of taxol.

The known methods for preparing the C-13 side chain moiety and its precursors all have serious disadvantages. In particular, they are uneconomical and unsuitable for such a preparation on a large scale.

For example, Holton in European Patent Application No. 0 400 971 A2 discloses taxol preparation by reacting β-lactams with an alcohol such as 7-0-triethylsilyl baccatin III in the presence of an activating agent to produce the corresponding β-amino ester. Thereafter, the triethylsilyl protecting group in the C-7 position and an ethoxyethyl protecting group in the C-2 position are hydrolyzed to restore the hydroxyl group in each position without disturbing the ester linkage or other taxol substituents. To prepare the substituted β-lactam, a racemic 3-hydroxy-4-arylazetidin-2-one is resolved by the formation of a diastereomeric ester (Mosher type) to obtain

a quantity of the pure dextrorotatory enantiomers prior to protection. Such a procedure is not only time consuming and labor intensive, but also inherently results in yields which are so low as to be commercially impractical.

Only one chemoenzymatic synthesis is now known to have been previously reported [Hönig et al., Tetrahedron, 46,3841 (1990)], and it involves an enzymatic resolution of an azide derivative of a C-13 side chain moiety precursor. However, this synthesis requires many tedious operations including protection and deprotection steps as well as the removal of the terminal carboxy ester group before a coupling to the C-13 position of a modified 10-deacetyl baccatin III can take place.

Consequently, both the Holton synthesis and the Honig et al. synthesis are impractical. Each synthesis provides yields of taxol that are impractical for commercial usage.

The art needs a new, improved and commercially practical method for preparing an intermediate for the C-13 side chain moiety and its precursors. Also, such methods of intermediate preparation should make possible new and useful techniques for making taxol. Further, such methods should also be capable of producing at practical cost and in practical quantities other similar and structurally related taxol-type compounds for purposes of research and development so that the pharmaceutical usage of taxol-type compounds can be more fully developed.

The present invention provides such preparation methods and also new intermediate enantiomers that are useful in methods for making taxol-type compounds.

The present invention provides a new and very useful process for preparing taxol-type compounds (including taxol itself) by coupling starting baccatin III-type compounds with side chain precursors for the taxol C-13 position. The present invention also provides methods for making such side chain precursors chemoenzymatically from certain readily synthesized β-lactams.

The present invention further provides both new and very useful intermediate enantiomers that are useful in the making of taxol-type compounds as well as the taxol-type compounds that are made therefrom.

These new preparation methods involve relatively few steps, are well-suited for sequential step utilization in commercial scale practice, and produce taxol-type compounds, including taxol itself, in relatively high yields and at relatively low cost.

For purposes of producing taxol, 10-deacetyl baccatin III itself is the preferred starting baccatin III-type compound. However, other starting baccatin III compounds can also be employed to make other taxol-type compounds according to the teachings of this invention.

An important aspect of this invention is the methodology that is provided for enzymatically making intermediate 1-substituted-β-lactam enantiomers that are useful as intermediates for making taxol-type compounds. The enantiomers are either (3R,4S) or (3S,4R) and they can be prepared in high yields. Either enantioselective hydrolysis or enantioselective transesterification using lipases is practiced. These β-lactam enantiomers can be directly coupled with baccatin III-type alcohol compounds (i.e., diterpene-type alcohols) to produce taxol-type compounds (including taxol).

If desired, these β-lactam enantiomers, can, either before or after cleaving, be subjected to one or more further synthetic step sequence(s) before being coupled to baccatin III-type compounds, thereby to introduce different functionalities into, or change functionalities existing in, the enantiomers or the cleaved products.

Substrate (starting) substituted β-lactam compounds for lipase treatment according to this invention are commonly racemic mixtures which can themselves be prepared synthetically in high yields from low cost starting materials using conventional methods.

The present invention, in one aspect, provides enzymatic methods for producing a substituted β-lactam enantiomer from a starting β-lactam racemate in high optical purity by treatment with a selected lipase in a suitable solvent to achieve at least a partial, and preferably a substantial, kinetic resolution of the starting compound. Such enantiomer can be converted into taxol-type compounds.

In one procedure, the lipase is employed, preferably under aqueous liquid phase conditions, to achieve enantioselective hydrolysis of an ester, thereby furnishing, for example, a preferred 3-hydroxy-4-aryl-β-lactam derivative in one absolute enantiomeric configuration, such as (3R,4S).

In another procedure, the lipase is employed, preferably under organic liquid phase conditions, to achieve the enantioselective transesterification of esters and yielding, for example, a preferred 3-hydroxy-4-aryl-β-lactam derivative in the opposite enantiomeric absolute configuration, such as (3S,4R).

In another aspect, new intermediate enantiomers and new taxol-type compounds are prepared. A presently preferred class of enantiomeric derivatives comprises (3R,4S) or (3S,4R)-3-hydroxy-4-aryl-β-lactams, and a presently most preferred enantiomer is a (3R,4S)-1-benzoyl-3-hydroxy-4-phenyl-β-lactam which, when coupled to derivatized 10-deacetyl baccatin III, yields taxol.

Certain members of the lipase family are now preferred for use in this invention.

One feature of lipases in the context of the present invention is that they can tolerate high substrate and

product concentrations in the liquid phase reaction medium. Thus, no marked substrate, and also no product, inhibition are generally observed. Also, the desired enzymatic reactions can be conducted with high substrate concentrations; and high enantioselectivity is easily achievable.

Another feature of lipases in the context of the present invention is their ease of usage. They can be immobilized and used in solution. Also, they can be recycled many times in view of their remarkable stability under the reaction conditions utilized herein.

Other and further objects, aims, features purposes, advantages, examples, embodiments, variations and the like will be apparent to those skilled in the art from the description in the present specification.

(a) Starting Materials

Starting alcohols useful in the practice of this invention have the diterpene ring structure_occurring in taxol or 10-deacetyl baccatin III and also a hydroxyl group in the C-13 position. Various substituents can be present at other positions, such as at C-1, C-2, C-3, C-7, C-8, C-10, C-12 and C-15; however, for reasons of availability and known or presently perceived pharmacological utility upon further reaction in the C-13 position, alcohols of the following generic formula are presently preferred:

(4)

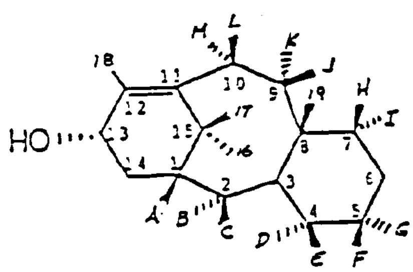

wherein:

A is a radical selected from the group consisting of hydrogen, hydroxyl, triethylsilyloxy, lower alkyl, lower alkenyl, lower alkoxy, lower alkanoyloxy, lower alkenoyloxy and aryloyloxy,

B and C are each an independently selected radical from the group consisting of hydrogen, hydroxyl, triethylsilyloxy, lower alkanoyloxy, lower alkenoyloxy, aryloyloxy and cyclohexoyloxy,

B and C taken together can form an oxo radical,

D is a radical selected from the group consisting of hydrogen, hydroxyl, lower alkanoyloxy, lower alkenoyloxy and aryloyloxy,

D and E taken together can form a radical selected from the group consisting of oxo and methylene,

E and F together with the carbon atoms between them can form an oxetane (i.e., an oxycyclobutyl) ring,

F and G are each an independently selected radical from the group consisting of hydrogen, hydroxyl, triethylsilyloxy, lower alkanoyloxy, lower alkenoyloxy, aryloyloxy and phen(lower alkan)oyloxy,

F and G taken together can form an oxo radical,

H and I are each an independently selected radical from the group consisting of hydrogen, hydroxyl, triethylsilyloxy, lower alkanoyloxy, lower alkenoxyloxy and aryloyloxy,

J and K are each an independently selected radical from the group consisting of hydrogen, hydroxyl, triethylsilyloxy, lower alkanoyloxy, lower alkenoyloxy and aryloyloxy,

J and K taken together can form an oxo radical,

L and M are each an independently selected radical from the group consisting of hydrogen, hydroxyl, triethylsilyloxy, lower alkanoyloxy, lower alkenoyloxy and aryloyloxy, and

L and M together can form an oxo radical.

Alcohols of the class shown in Formula (4) have previously been identified as being useful in making taxol-type compounds; see, for example, Holton U.S. Patent No. 5,015,744 and Holton EPA 0 400 971 A2.

Presently preferred compounds of Formula (4) are 7-O-triethylsilyl baccatin III derivatives as shown in Formula (5) below:

(5) 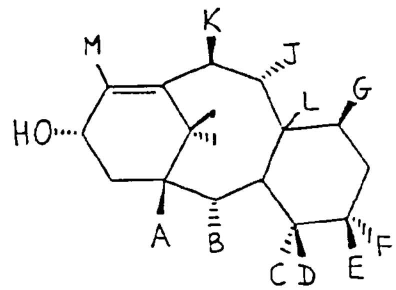

where:

A is selected from the group consisting of hydrogen, hydroxyl and lower alkyl,

B is selected from the group consisting of hydrogen, hydroxyl, lower alkanoyloxy, benzoyloxy, cyclohexoyloxy and triethylsilyloxy,

B together with an adjacent bond on the same carbon atom can form an oxo (= O) group,

C is selected from the group consisting of lower hydroxyl and alkanoyloxy,

C and D together can form an oxo-group,

D and E together with the carbon atoms between them can form an oxetane (i.e., an oxycyclobutyl) ring,

F is selected from the group consisting of hydrogen, hydroxyl, triethylsilyloxy and phen(lower alkan)oyloxy,

G is hydrogen, hydroxyl and triethylsilyloxy,

J is selected from the group consisting of hydrogen, hydroxyl, triethylsilyloxy, lower alkanoyloxy,

J together with an adjacent bond on the same carbon atom can form an oxo group,

K is selected from the group consisting of hydroxyl, triethylsilyloxy and lower alkanoyloxy,

K together with an adjacent bond on the same carbon atom can form an oxo group,

L is selected from the group consisting of hydrogen and methyl, and

M is selected from the group consisting of hydrogen and methyl.

The 7-O-triethylsilyl group is a protective group to protect the C-7 hydroxyl group during esterification of the C-13 hydroxyl group as described herein.

Greene et al. describes the conversion of 10-deacetyl baccatin III to a taxol intermediate by the attachment of an acetyl group thereto at the C-10 position and by the attachment of a protective O-triethylsilyl group containing side chain at the C-7 position. The hydroxyl group at the C-13 position is then coupled to a compound having a β-amido ester group to achieve a taxol-type compound. Although, as in Greene et al., it is presently most preferred to have an acetyl group at the C-10 position, those skilled in the art will appreciate that other higher alkanoyloxy substituents can be placed at the C-10 position by essentially the same reaction that is described by Greene et al. by substituting a corresponding lower alkanoyl chloride for the methanoyl chloride that is used with pyridine by Greene et al. These higher alkanoyloxy substituents may produce useful pharmaceutically active taxol-type compounds. The starting alcohols of Formula (4) are not limited to C-10 acetyl groups for purposes of practicing the processes of this invention, as those skilled in the art will appreciate.

Various examples of compounds of Formulas (4) and (5) are shown in Table I below:

Table I

| I.D. No. | Exemplary Starting Alcohols | Preparation |
|---|---|---|
| 4.1 | 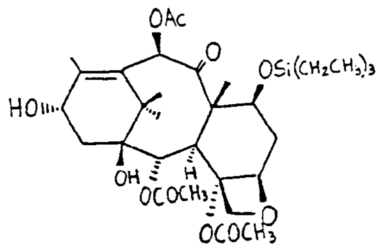 | F.N. 4.1 |
| 4.2 | 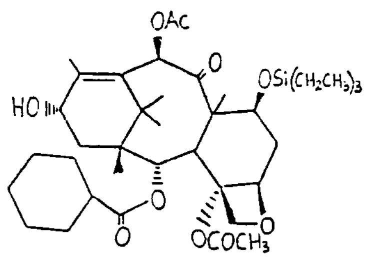 | F.N. 4.2 |
| 4.3 | 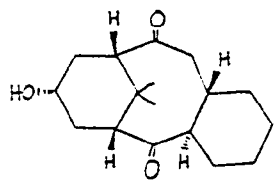 | F.N. 4.3 |
| 4.4 | 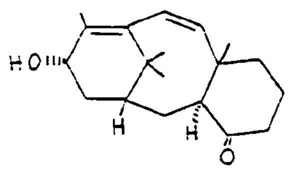 | F.N. 4.4 |
| 4.5 | 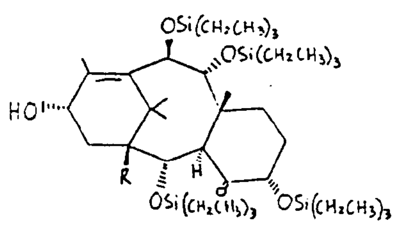 | F.N. 4.5 |
| 4.6 | 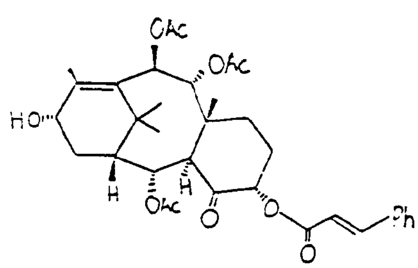 | F.N. 4.6 |

| I.D. No. | Exemplary Starting Alcohols | Preparation |
|---|---|---|
| 4.7 | 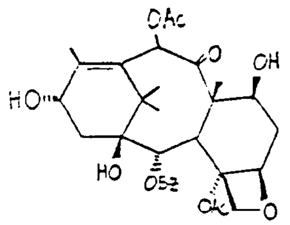 | F.N. 4.7 |
| 4.8 | 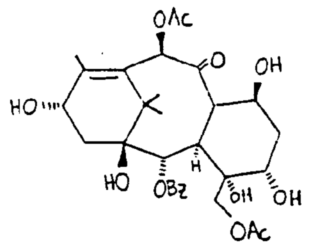 | F.N. 4.8 |
| 4.9 | 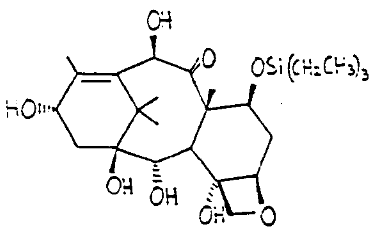 | F.N. 4.9 |

Table I Footnotes:

| | |
|---|---|
| 4.1 | 10-deacetyl baccatin III is reacted with triethylsilyl chloride to provide the starting alcohol, 7-O-triethylsilyl-10-deacetyl baccatin III. Greene et al., J. Am. Chem. Soc., 110, 5917 (1988). |
| 4.2 | This alcohol is disclosed by Kingston et al., J. Nat. Prod. 53, 1-2 (1990); see structure 19. |
| 4.3 | This alcohol is disclosed by Blechert et al., Angew. Chem. Int. Ed. Engl., 30, 412 (1991). |
| 4.4 | This alcohol is disclosed by Kende et al., J. Am. Chem. Soc., 108, 3513 (1986). |
| 4.5 | This alcohol is disclosed by Chan et al., J. Am. Chem. Soc., (Chem. Commun.), 88, 923 (1966). |
| 4.6 | This alcohol is disclosed by Shiro et al., J. Am. Chem. Soc., (Chem. Commun.), 88, 98 (1966). |
| 4.7 | This alcohol is disclosed by Magri et al., J. Org. Chem., 51, 3239 (1986). |
| 4.8 | This alcohol is disclosed by Samaranayake et al., J. Org. Chem., 56, 5114 (1991). |
| 4.9 | This alcohol is disclosed by Kingston et al., J. Nat. Prod., 53, 1-2 (1990); see structure 20. |

Other and further examples of suitable starting alcohols of Formula (4) will be apparent to those skilled in the art from the descriptions of the present invention and from the prior art. Tertiary alcohols are more difficult to form into ethers than primary or second alcohols, especially when they are hindered at the C-1 and C-4 positions. The silyl groups are easily removed, as described by Holton, ibid.

One can selectively block positions C-2 and C-10 by acylation or ether formation. Greene et al., J. Am. Chem. Soc., 110, 5917 (1988), describes the selective acylation at C-10. One can form an ether selectively at C-2 using a variety of reagents. The formation and removal of ethers is comprehensively described in "Protection Groups In Organic Synthesis", T.W. Greene, John Wiley & Sons, pp. 14-50 (1981).

A starting β-lactam useful in the practice of the methods of this invention can have any one of a wide variety of structures. One presently preferred and exemplary starting β-lactam class is characterized by the generic formula:

(6)

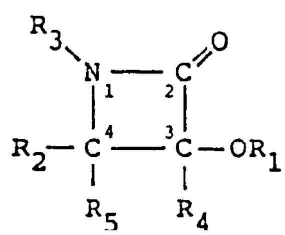

where:

$R_1$ is a radical selected from the group consisting of hydrogen, lower alkanoyl, lower α-haloalkanoyl and lower alkenoyl,

$R_2$ is a radical selected from the group consisting of hydrogen, aryl, substituted aryl, aralkyl, lower alkyl and lower alkenyl,

$R_3$ is a radical selected from the group consisting of hydrogen, hydroxyl, lower alkanoyl, lower alkenoyl and aryloyl,

$R_4$ is a radical selected from the group consisting of hydrogen, lower alkyl and lower alkenyl, and

$R_5$ is a radical selected from the group consisting of hydrogen, lower alkyl and lower alkenyl.

The term "lower" as used herein to describe an organic moiety indicates that such moiety contains less than about seven carbon atoms.

As used herein, the term "aryl" alone or with a prefix or suffix refers to an aromatic ring structure containing or incorporating 6 through about 12 carbon atoms.

More preferably in Formula (6),

$R_1$ is a radical selected from the group consisting of hydrogen, lower alkanoyl and lower $\alpha$-haloalkanoyl,

$R_2$ is a radical selected from the group consisting of phenyl, lower alkyl phenyl, and methoxyphenyl,

$R_3$ is a radical selected from the group consisting of hydrogen, benzoyl, methoxyphenyl, and t-butanoyl, and

$R_4$ and $R_5$ are each an independently selected radical selected from the group consisting of hydrogen and lower alkyl.

A preferred "halo" substituent is chloro.

In general, for purposes of carrying out enantioselective hydrolysis in accord with this invention, $R_1$ in Formula (6) should preferably be an ester group, such as defined above in Formula (6) since a hydroxyl group in the C-3 position of a starting β-lactam is acted upon by a lipase enzyme or the like. For purposes of carrying out enantioselective transesterification in accord with this invention, $R_1$ in Formula (6) should preferably be hydrogen.

A presently preferred starting β-lactam for use in lipase-catalyzed enantioselective hydrolysis in accord with the present invention is a β-lactam within the scope of Formula (6) which has the following generic formula:

(7)

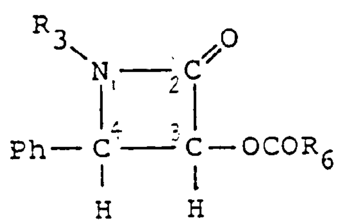

where:

$R_3$ is a radical selected from the group consisting of hydrogen, p-methoxyphenyl, t-butanoyl and benzoyl,

$R_5$ is a radical selected from the group consisting of hydrogen and lower alkyl, and

Ph is phenyl.

A presently preferred starting β-lactam for use in lipase-catalyzed enantioselective transesterification in accord with the present invention is a β-lactam within the scope of Formula (6) which has the following generic formula:

(8)

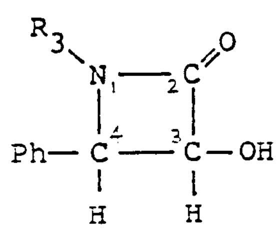

where:

Ph is phenyl, and

$R_3$ is a radical selected from the group consisting of hydrogen, benzoyloxy, methoxyphenyl and t-butanoyloxy.

Characteristically and typically, a starting β-lactam of Formula (6), (7) or (8) is a racemic mixture of position isomers (including optically active isomers). Preferably, a particular starting β-lactam is a substantially pure

racemic mixture; that is, the starting β-lactam contains only one β-lactam compound having one particular substituent for each of the substituents $R_1$ through $R_5$.

The starting substituted β-lactam (also known as azetidin-2-ones) of Formulas (6), (7) and (8) are prepared by various known synthetic procedures. Also, various such compounds can be prepared by converting starting β-lactams which are readily available commercially from various suppliers of organic chemicals.

For example, the preparation of 1-p-methoxyphenyl-3-acetoxy-4-phenylazetidin-2-one is described by Holton on page 14 of EPA 0 400 971 A2. This compound is oxidizable as described by Holton using ceric ammonium nitrate to 3-acetoxy-4-phenylazetidin-2-one. In turn, this last named product is catalytically converted to 1-benzoyl-3-acetoxy-4-phenyl-azetidin-2-one using 4-dimethylaminopyridine and triethylamine as described by Palomo et al. [Tetrahedron Letters, 31, 6429-6432 (1990)], and this last named product is reductively converted to 1-hydroxyl-3-acetoxy-4-phenyl-azetidin-2-one using potassium hydroxide and tetrahydrofuran.

β-Lactams with other or additional substitutes in the C-4 position can be prepared by initially selecting an appropriate combination of substituted aniline with a lower alkyl or a lower alkenyl substituted amine for use in the starting β-lactam formation method described by Holton, ibid. The foregoing illustrative β-lactam preparation procedures are summarized as follows:

(9)

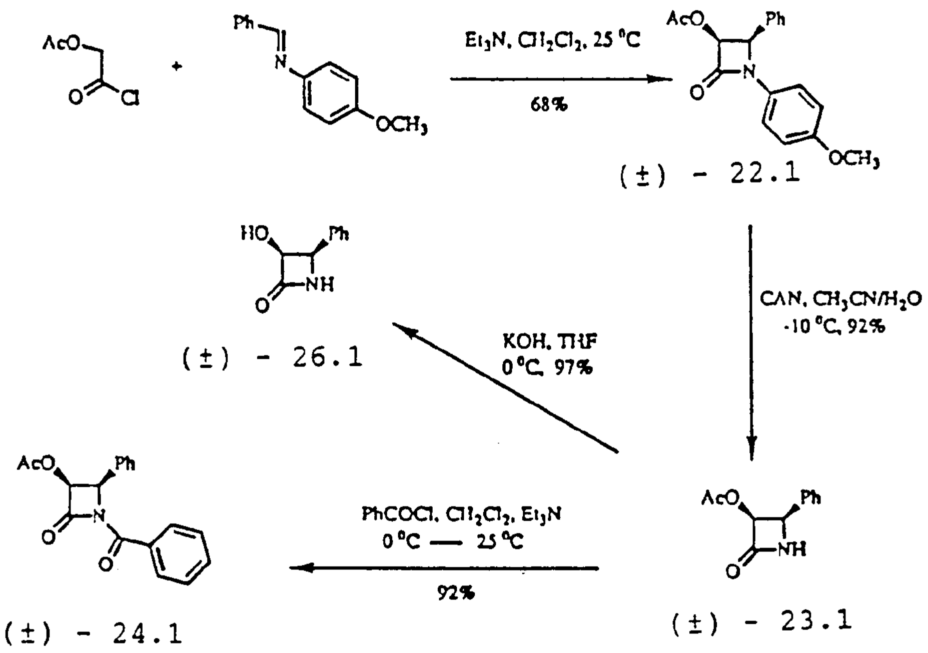


(±) - 22.1

(±) - 23.1

(±) - 24.1

(±) - 26.1

When the lipase-catalyzed enantioselective transesterification of this invention is practiced (as described herein), a starting 1-(substituted) ethenyl acetate is employed which is preferably characterized by the generic formula:

(10)

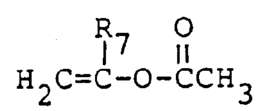

where $R_7$ is a radical selected from the group consisting of hydrogen and lower alkyl.

A presently most preferred ester of Formula (10) is vinyl acetate (where $R_7$ is hydrogen).

Lipases useful in the practice of this invention are preferably characterized by the capacity to produce either enantioselective hydrolysis or enantioselective transesterification, as desired, of a particular starting β-lactam of Formula (6), (7) or (8).

Preferably, the lipase used is enantioselective to the extent that it produces a substantial enantiomeric excess of a 3-hydrcxyl-β-lactam enantiomer, such as an excess of at least about 95%. Examples of suitable lipases include the Pseudomonas and the like.

For hydrolysis, the particular preferred lipases are identified herein as P-30, K-10, AK, AP, PL, CCL, PPL and FAP. For transesterification, the presently preferred lipases are identified as P-30 and AK. Presently most

preferred lipases are identified as P-30 and AK which belong to the bacterial lipases of the genus Pseudomonas. These lipases are all obtainable from commercial sources including Amano International Enzyme, Troy, VA as described herein. Starting lipases in a crude (i.e., impure) form are convenient to use, but preferably a starting crude lipase has a purity of at least about 1% with the remaining components being substantially inactive (i.e., inert) when in use in the practice of this invention. It is presently preferred to employ lipases which are characterized by the capacity to produce (when contacted with a starting β-lactam of Formula (6) in accord with the teachings of this invention) an enantiomeric excess of an enantiomeric product of Formula (17) or of Formula (18) (below) which excess is at least about 100 times greater than the amount of other β- lactam product compounds concurrently produced during the contacting.

Conventional phosphate buffers are employed in the practice of this invention such, for example, as $Na_2HPO_4/NaH_2PO_4$, $K_2HPO_4/KH_2PO_4$ or the like. One presently preferred phosphate buffer is 0.2M $Na_2HPO_4/NaH_2PO_4$.

Emulsifiers are optional but preferred starting materials for use in a liquid reaction medium used in the practice of this invention. Examples of suitable and presently preferred emulsifiers include polyvinyl alcohol, propylene glycol, and the like.

(b) Preparation of β-Lactam Enantiomers

To produce a substituted β-lactam enantiomer, one contacts a lipase as characterized above with a starting β-lactam with a compound of Formula (6), preferably under liquid phase conditions.

Optionally, bit preferably, the starting β-lactam and the lipase are both dissolved in the liquid phase medium. However, the enantioselective conversion process that occurs will take place if the starting β-lactam and/or the lipase are only suspended in the liquid medium; for example, as colloidal particles.

Optionally, but preferably, the liquid medium further includes dissolved therein an emulsifier such as characterized above.

When enantioselective hydrolysis is being carried out, the liquid medium is preferably aqueous. When enantioselective transesterification is being carried out, the liquid medium is preferably organic and an ester of Formula (10) is additionally present in the liquid medium, preferably in dissolved form.

When the liquid medium is aqueous, a phosphate buffer is preferably included such as described above.

Preferably, the contacting is carried out until at least about 50 weight percent of the starting β-lactam has been enzymatically converted into enantiomeric product compounds, although higher and lower starting material conversions can be employed.

The contacting is also preferably carried out at a temperature in the range of about 10°C to about 50°C, although higher and lower temperatures may be used. Presently most preferred contacting temperatures are about 25°C for hydrolysis, and about 50°C for transesterification. The temperature and pressure conditions of contacting are interdependent, as those skilled in the art will appreciate. Atmospheric pressure is presently preferred, but higher and lower pressures can be employed, if desired.

Preferably, the contacting is carried out at a pH of the liquid medium that is in the range of about 3 to about 10, although higher and lower pH values may be employed. In the case of an aqueous medium, the pH is more preferably in the range of about 7 to about 8. A presently most preferred aqueous pH is about 7.5.

The starting β-lactam preferably has a concentration in the liquid medium that is in the range of about 0.1 to about 1 molar (M), although higher and lower molar concentrations may be employed.

Preferably, the weight ratio of the starting β-lactam to the starting lipase is in the range of about 1:1 in the liquid medium, although higher and lower weight ratios can be employed.

The liquid medium in which the contacting is conducted can be either aqueous or organic. Mixtures of aqueous and organic liquids can be used. For convenience, the term "aqueous medium" is used to mean a liquid which comprises at least about 50 weight percent water (with the balance up to 100 weight percent of the medium being organic liquid), while, correspondingly, the term "organic medium" is used to mean a liquid which comprises at least about 50 weight percent organic liquid (with the balance up to 100 weight percent of the medium being water).

An organic liquid or an organic medium should preferably be not only a solvent for the lipase and the starting β-lactam, but also non-lipase denaturing. Many different organic liquids are suitable. Examples include carbon tetrachloride, cyclohexane, carbon disulfide, hexane, acetonitrile, and the like. Acetonitrile is presently a most preferred organic liquid.

The starting β-lactam can be added to a liquid reaction medium in a solid or in a liquid form (for example, in an aqueous solvent or the like).

The contacting can be monitored, preferably continuously, for example, by thin layer chromatographic analysis of the liquid reaction medium using a convenient developing solvent, such as a 1:1 weight ratio mixture

of ethyl acetate and hexane or the like.

Any convenient means or procedure can be used to terminate the contacting. One presently preferred procedure is to extract the liquid reaction medium with a suitable non-medium miscible solvent after removal of the lipase therefrom. One suitable and presently preferred solvent for such an extraction is ethyl acetate.

The contacting causes an enzymatic reaction to occur in which the substrate is structurally altered and converted into two different classes of chiral product β-lactam compounds. In one of these product classes, the compounds are substituted β-lactam enantiomers of one absolute configuration, while, in the other (second) one of the product classes, the compounds are substituted β-lactam enantiomers of the opposite configuration.

Thus, one product class of compounds has the configuration (3R,4S) while the other (second) product class of compounds has the configuration (3S,4R). Furthermore, the compounds of one product class have hydroxyl substitution in the C-3 position on the β-lactam ring while the compounds of the other (second) product class have an ester group in the C-3 position on the β-lactam ring.

Which one of the two product classes has C-3 position hydroxyl substitution depends upon whether hydrolysis or transesterification is accomplished during the contacting. Thus, when the selected lipase enzyme is used to catalyze the enantioselective hydrolysis of an ester group existing in the C-3 position of a starting β-lactam, then the ester moiety initially existing in the C-3 position of the starting β-lactam is converted to an hydroxyl group. However, when the same or different selected lipase enzyme is used to catalyze enantioselective transesterification of the moiety existing in the C-3 position of a starting β-lactam (preferably an hydroxyl group, but an ester group can also be initially at this C-3 position), then that initial moiety is converted to a (different) ester group.

When enantioselective hydrolysis is carried out, the members of the class of the chiral product compounds produced which have an hydroxyl group in the C-3 position of the β-lactam ring have the (3S,4R) configuration. The members of the other (second) class of chiral product compounds produced have an ester group in the C-3 position of the β-lactam ring have the (3R,4S) configuration. This hydrolysis synthesis is illustrated by the following equation (where the starting substituted β-lactam is illustratively a Formula (7) compound):

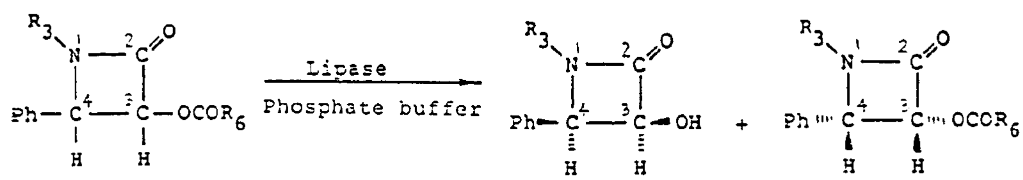


(11)

Formula (7) (3S,4R) (3R,4S)

Formula (11.1) Formula (11.2)

When enantioselective transesterification is carried out, the selected enzyme is used in an organic liquid medium which is preferably substantially anhydrous. However, the enzyme must contain at least about 3% of core water of maintain the protein in the proper conformation. An ester of Formula (10) is preferably dissolved additionally in the liquid medium (along with the lipase and the starting β-lactam). The mole ratio of the starting β-lactam to the starting ester of Formula (8) is preferably in the range of about 1 to about 2 or 3, although higher and lower mole ratios can be employed. Here, one of the two chiral product compound classes produced has member compounds with a hydroxyl group in the C-3 position of the β-lactam ring and the (3R,4S) configuration, while the other (second) chiral product compound class produced has compounds with an ester group in the C-3 position of the β-lactam ring and the (3S,4R) configuration. This synthesis is illustrated by the following equation (where the starting β-lactam is illustratively a Formula (8) compound):

(12)

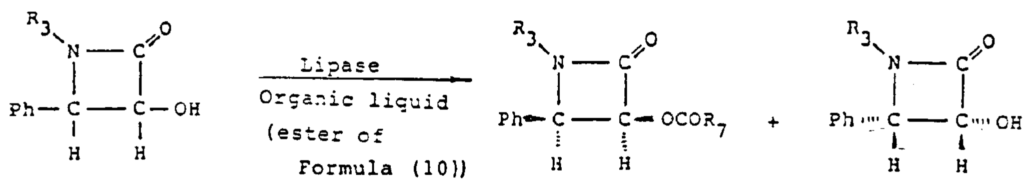


Formula (8) (3S,4R) (3R,4S)

Formula (12.1) Formula (12.2)

In these enantioselective enzymatic reactions, as indicated above, it is preferred to employ lipases which produce an enantiomeric ratio (E) of the product class having hydroxyl substitution in the C-3 position of the β-lactam ring to the remaining substrate having ester substitution in the C-3 position of the β-lactam ring that is in excess of about 75 and that more preferably is in excess of about 100. For present purposes, the enantiomeric ratio (E) is calculated from the relationship:

$$(13) \qquad E = \frac{\ln[(1-c)(1-ee_S)]}{\ln[(1-c)(1+ee_S)]}$$

wherein c is the extent of conversion.

The value of c is given by the expression:

$$(14) \qquad c = \frac{ee_S}{ee_S + ee_P}$$

where, for hydrolysis:

$ee_s$ is the enantiomeric excess of the remaining substrate (ester), and

$ee_p$ is the enantiomeric excess of the product (alcohol); and

where, for transesterification:

$ee_s$ is the enantiomeric excess of the remaining substrate (alcohol), and

$ee_p$ is the enantiomeric excess of the product (ester).

The relationship and the measuring techniques involved are described by Chen et al. in J. Am. Chem. Soc., 104, 7294 (1982).

The preferred lipases indicated above produce the preferred enantiomeric ratios (E).

Lipases have various features and advantages. One feature of lipases is that they can tolerate high substrate and high product concentrations in the liquid reaction medium. Thus, no marked substrate or product inhibition is generally observed. Also, the enzymatic reactions can be conducted with high substrate concentrations. For example, substrate concentrations in the range of about 0.1 to about 1 M, and a high degree of enantioselectivity can be achieved.

Moreover, the lipases may be immobilized in solution. For example, the lipases can be immobilized by adsorption on "Celite" (a trademark of Manville Products Corporation for its brand of diatomaceous earth). For example, the commercially available Pseudomonas cepacia P-30 (Amano) lipase (2 g) was dissolved in 4 ml of deionized distilled water and 2 g of Celite was added. After thorough mixing by stirring, the mixture was lyophilized under high vacuum for 20 hours (-78°→25°C) using a cryolizer-type freeze drying apparatus to provide 3.91 g of a solid powder. The water content of the preparation was around 2.7%.

Also, the lipases may be recovered and recycled by reincubation many times because of their remarkable stability under the reaction conditions described herein.

A resulting substantially lipase-free organic liquid reaction medium is conveniently dried over a drying agent, such as anhydrous magnesium sulfate or the like, and is conveniently evaporated to dryness in vacuo.

Thereafter, the recovered mixture (ester and alcohol) is subjected to separation using column chromatography or other suitable or conventional procedure. Any convenient method of recovery and purification can be employed.

After separation of an enantiomer by, for example, column chromatography (using, for example, a procedure such as described in Example 1 below), rotation is conveniently determined in trichloromethane or methanol using a Perkin-Elmer Model 241 polarimeter or the like. The enantiomeric excess is conveniently determined by proton NMR in the presence of a chiral shift reagent such as $Eu(hfc)_3$.

Thus, a wide variety of substituted β-lactam enantiomers can be produced by the process of this invention. Presently preferred substituted β-lactams are the enantiomers of Formulas (11.2) and (12.2).

Presently most preferred enantiomer products are β-lactam enantiomers which are characterized by the formulas:

(15)

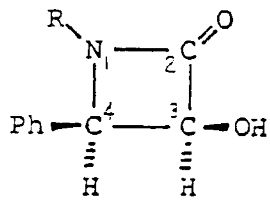

(3S,4R)

and

(16) 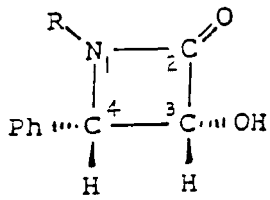

(3R,4S)

where, in each of Formulas (15) and (16):

Ph is phenyl, and

R is selected from the group consisting of hydrogen, benzoyl, t-butanoyl and methoxyphenyl.

Thus, in general, by contacting starting racemic Formula (6) compounds with enantioselective lipases using either conditions of hydrolysis or of transesterification followed by separation as described by the present invention, two different classes of substituted β-lactam enantiomers are produced. One class has the non-natural (3S,4R) structure and is characterized by the generic formula:

(17) 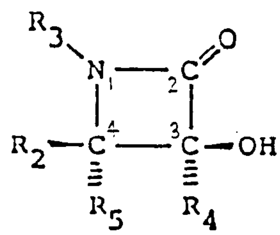

The other class has the natural (3R,4S) structure and is characterized by the generic formula:

(18) 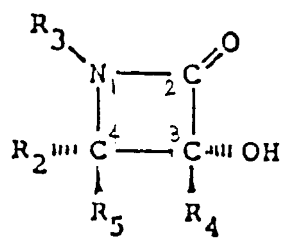

In Formulas (17) and (18), $R_2$, $R_3$, $R_4$ and $R_5$ are as defined in Formula (6).

The class of enantiomeric compounds of Formula (17) is believed to be new, and, so far as now known, these compounds have not previously been prepared in a purified form.

Although some of the enantiomeric compounds of Formula (18) were previously disclosed (see Holton in EPA 0 400 971 A2) as being capable of preparation by diastereomeric ester formation, followed by recrystallization, those enantiomer compounds of Formula (18) wherein $R_4$ and $R_5$ are each independently selected from the group consisting of lower alkyl and lower alkenyl are believed to be new. So far as now known, such compounds have not previously been prepared in a purified form, and these compounds cannot currently be readily prepared in commercial quantities by conventional procedures.

An enantiomer of either Formula (17) or of Formula (18) is coupled to a starting alcohol of Formula (4) to produce a taxol-type compound.

(c) <u>β-Lactam Coupling to Alcohols and Taxol-Type Compounds</u>

The coupling reaction is conveniently carried out by contacting such an enantiomer (suitably protected) with such an alcohol (suitably protected) in the presence of an activating agent at ambient temperatures. Preferably, the activating agent is a tertiary amine, such as 4-dimethylaminopyridine, pyridine, triethylamine, diisopropyl ethyl amine, N-methyl imidazole or the like.

Preliminary protection of reactive sites either on the starting alcohol or on the starting β-lactam enantiomer (which sites are not to be disturbed during the coupling reaction) can be conventionally achieved with protecting groups, as those skilled in the art will readily appreciate (see, for example, Greene et al., ibid.,in "Protection Groups in Organic Synthesis"). Various protecting groups are suitable. Ethoxyethoxy and triethylsiloxy protecting groups are presently preferred and are illustrated in the following Examples.

During the coupling reaction, a selected enantiomer of either Formula (17) or Formula (18) cleaves and the β-lactam forms an ester linkage with the C-13 hydroxyl group of the Formula (4) alcohol.

After the coupling reaction, protecting groups that remain associated with the product ester compound are hydrolyzed under mild conditions so as to avoid disrupting the ester linkage or other substituents. This hydrolyzing, for example, can be carried out with HCl and an ethanol/water mixture at ambient temperatures.

After the coupling reaction (and before a hydrolyzing reaction if such a hydrolyzing reaction is needed because of the presence of protecting groups), the product ester can be and preferably is separated and purified. For example, a solvent such as ethyl acetate or the like can be added at the end of the coupling reaction and the resulting solution can be washed with aqueous sodium bicarbonate solution, dried over magnesium sulfate, and concentrated (such as by vacuum evaporation). This concentrate can be filtered through silica gel, for example, and then eluted with ethyl acetate or the like. The residue can then be purified by flash chromatography and eluted with a solvent mixture, such as ethyl acetate and hexane. A subsequent recrystallization from ethyl acetate or the like can be used to produce an enantiomeric product that can then be hydrolyzed.

After a hydrolyzing reaction (or hydrolysis), the product can be separated and purified. For example, ethyl acetate or the like can be used as the solvent, and the solution can be extracted with aqueous sodium bicarbonate solution, dried over sodium sulfate, and concentrated. The residue can be purified by column chromatography on silica gel eluted with, for example, a mixture of ethyl acetate and hexane.

Preferably, the starting alcohol for the coupling reaction is a Formula (5) compound. Thus, a present particularly preferred starting alcohol type is a 7-0-triethylsilyl baccatin III-type compound in which the C-7 and C-2 hydroxyl groups are protected with triethylsilyl and ethoxyethyl protecting groups, respectively. A presently most preferred starting alcohol of Formula (5) is 7-0-triethylsilyl baccatin III.

As those skilled in the art will appreciate, to produce from such a coupling reaction taxol-type compounds which have a C-13 side chain that has the naturally occurring (3R,4S) structure with respect to its C-2' and C-3' carbon atoms (comparable to taxol itself), a β-lactam of Formula (18) (suitably protected) is employed. However, when a β-lactam of Formula (17) (suitably protected) is employed alternatively in such a coupling reaction, a class of non-naturally occurring taxol-type compounds is produced wherein the C-13 side chain has the (3S,4R) structure. So far as now known, compounds of the latter class have not previously been known or produced, and only a certain few compounds of the former class have previously been known or produced (now believed to be taxol-type compounds (including taxol) wherein, in Formula (17) β-lactams, $R_4$ and $R_5$ are each hydrogen).

To produce taxol from a Formula (18) β-lactam having the natural (3R,4S) structure by a coupling reaction with 7-O-triethylsilyl baccatin III, various β-lactams of Formula (18) can be used. For one example, and as specifically described in Example 18 below, cis-3R-hydroxy-4S-phenylazetidin-2-one (the compound of Formula (26.2) is first protected at the 3-hydroxyl group by converting this group to a 1-ethoxyethoxy (EEO) group (Formula 27.1) and then the ring 1-nitrogen has its hydrogen replaced by a benzoyl group to produce cis-1-benzoyl-3R-(1-ethoxyethoxy)-4S-phenylazetidin-2-one (Formula 28). This product is then coupled with the 7-O-triethylsilyl baccatin III and then hydrolyzed to produce taxol.

For another example, and as specifically illustrated in Example 19 below for the corresponding non-natural-type cis-1-benzoyl-3S-hydroxy-4R-phenylazetidin-2-one from Formula (18), the natural-type compound cis-1-benzoyl-3R-hydroxy-4S-phenylazetidin-2-one of Formula (17) is first protected at the 3-hydroxyl group with a triethylsiloxy group and this product is then coupled with 7-O-triethylsilyl baccatin III and hydrolyzed to produce taxol.

Other procedures for preparing taxol from (3R,4S) β-lactams of Formula (18) with 7-O-triethylsilyl baccatin III will be apparent to those skilled in the art.

Natural-type taxol-type compounds produced by the practice of the present invention incorporate a C-13 side chain which has the naturally occurring (3R,4S) structure. Such compounds are represented by the formula:

(19)

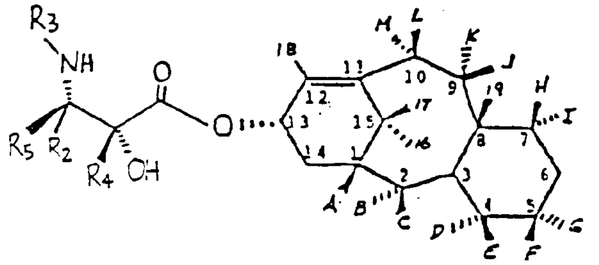

where

A through M each have their respective above-defined meanings as above defined in relation to Formula (4), and

$R_2$ through $R_5$ each have their respective above-defined meanings as above defined in relation to Formula (4).

Enantiomeric taxol-type compounds of Formula (19) where $R_4$ and $R_5$ in the C-13 side chain are each independently selected from the group consisting of lower alkyl and lower alkenyl are novel and presently preferred. These novel taxol-type compounds were not previously preparable particularly in a purified form because the corresponding intermediate enantiomeric β-lactams of Formula (18) (or other intermediate enantiomers) used in making them were either not known or could not be practically prepared by prior art procedures (as indicated above). These novel taxol-type compounds are believed to display pharmacological activities and to be useful for anti-cancer therapy in applications similar to those in which taxol and other taxol-type compounds wherein $R_4$ and $R_5$ are hydrogen have been found to be useful.

Non-natural-type taxol-type compounds produced by the practice of the present invention incorporate a C-13 side chain which has the non-naturally occurring (3S,4R) structure. Such compounds are represented by the formula:

(20)

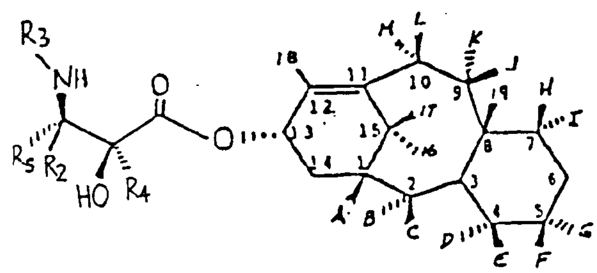

where each term has its respective above-defined meaning as in Formula (19).

The enantiomeric taxol-type compounds of Formula (20) are believed to be new. However, compounds of Formula (20) wherein $R_4$ and $R_5$ are each either lower alkyl or lower alkenyl are presently much preferred. Such compounds (particularly the indicated preferred compounds) have not previously been the prepared in purified form because the intermediate enantiomeric β-lactams of Formula (17) (or other enantiomeric intermediate) were either not known in, or could not be practically prepared by, prior art teachings. Compounds of Formula (20) are now believed to display pharmacological activities and are presently believed to be useful for anti-cancer therapy in applications similar to those in which taxol has been found to be useful.

Presently preferred compounds of each of Formulas (19) and (20) are those produced by using an alcohol of Formula (5). Thus, presently preferred novel taxol-type compounds of the invention are prepared as described herein by coupling a suitably protected Formula (5) alcohol with a suitably protected Formula (17) or Formula (18) substituted β-lactam having the natural (3R,4S) structure or the non-natural (3S,4R) structure wherein $R_2$ and $R_3$ are as above defined and $R_4$ and $R_5$ are each independently selected from the group consisting of lower alkyl and lower alkenyl. Such taxol-type compounds are characterized by having one of the following generic formulas:

(21A)

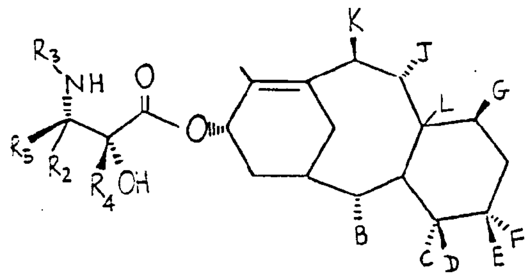

(21B)

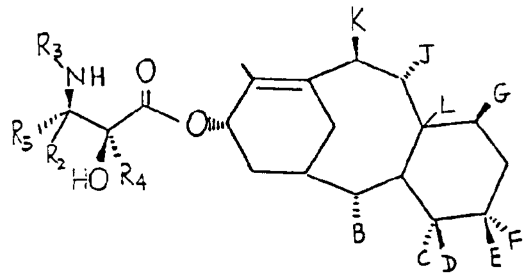

where:

$R_2$, $R_3$, $R_4$ and $R_5$ are as above defined in relation to Formula (6), and

A, B, C, D, E, F, G, J, K, L and M are as above defined in relation to Formula (5).

As indicated above, an enantiomer of either Formula (17) or of Formula (18) can be, if desired, further reacted before being coupled to a starting alcohol of Formula (4). Such a procedure can be used to change or alter substituents either on the β-lactam ring or on the subsequently formed C-13 side chain of a taxol-type compound.

As used herein, the term "alkyl" (or the equivalent) includes straight or branched monovalent chain radicals and includes methyl (presently preferred), ethyl, propyl, isopropyl, butyl, isobutyl, tert-butyl, hexyl, and the like.

As used herein, the term "alkenyl" (or the equivalent) refers to a monovalent radical of at least two carbon atoms with a single double band between a pair of adjacent carbon atoms. An alkenyl radical can be a straight or a branched chain and includes ethenyl, propenyl, isopropenyl, butenyl, isobutenyl, hexenyl, and the like.

As used herein, the term "alkanoyloxy" includes acetate, propionate, butyrate, valerate, isobutyrate and the like. Acetate is presently preferred.

Exemplary aryl or aryl containing radicals include phenyl (presently preferred) and naphthyl (α or β). Substituents include alkanoxy, hydroxy, halogen (preferably chloro), lower alkyl, lower alkenyl, lower acyl, lower acyloxy, nitro, amino, amido, and the like. Aromatic heterocyclic structures are included.

The following Examples are presented by way of illustration only and are not in any way to be construed as limiting the scope of the invention defined in the appended claims.

(a) Preparation of β-Lactam Enantiomers

Examples 1-7 Enzymatic enantioselective hydrolysis of (±)-cis-1-p-methoxyphenyl-3-acetoxy-4-phenylazetidin-2-one (16.1).

A representative equation for this hydrolysis is as follows:

(22)

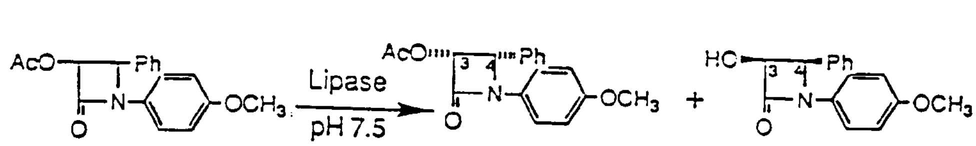


(±)-(22.1) (3R,4S)-(22.2) (3S,4R)-(22.3)

The racemate of Formula (22.1) is prepared as described by Holton on page 14 of EPA 0 400 971 A2.

To a suspension of Pseudomonas cetacia lipase (80mg) (Amamo P-30) in 3.6 ml of 0.2M aqueous phosphate buffer, pH 7.5, was added 100 mg of the substrate of Formula (22.1), dissolved in 0.4 ml of acetonitrile (cosolvent). The reaction mixture was stirred with a magnetic stirrer until about 50% conversion was reached

(25°C, 23 hrs.). The progress of the reaction was monitored by thin layer chromatography (TLC). The remaining substrate has an Rf value of 0.58 using ethyl acetate:hexane (1:1) as the eluent. The reaction was terminated by three successive extractions of the reaction medium each with ethyl acetate (3 x 25 ml), and the substrate and products were separated from the resulting liquid medium by silica gel flash chromatography using ethyl acetate:hexane (1:3 to 1:1). The optical purity of the remaining substrate expressed as enantiomeric excess (ee) was determined by $^1H$ NMR ($CDCl_3$, 200 MHz) analysis of the methoxy signal in the presence of the chiral shift reagent $Eu(hfc)_3$.

The product of Formula (22.3) was first converted into the product of Formula (22.2) by treating with acetic anhydride:pyridine (1:1) in the presence of-a catalytic amount of 4-dimethylaminopyridine and the optical purity was then analyzed similarly. The results are shown in Table II below.

Additional Examples were similarly carried out at 25°C and the results are also presented below in Table II. In Tables II and III, AK and K-10 refer to lipases of the Pseudomonas sp. which were obtained from Amano International Enzyme, Troy, VA; P-30 refers to the lipase derived from Pseudomonas cepacia which was obtained from Amano; PPL refers to porcine pancreatic lipase obtained from Sigma Chemical Co., St. Louis, MO; AY-50 refers to Candida cylindracea obtained from Amano; FAP refers to Rhizopus javanicus obtained from Amano; AP refers to Amano AP (Aspergillus niger) sold by Amano; AY refers to Candida cylindracea obtained from Amano; PL (Meito) refers to Alcaligenes sp. obtained from Meito Sangyo Ltd., Tokyo, Japan; Lipase (Seik) refers to Rhizpus delemar obtained from Seikagaku Kogyo Co.,; MY and CCL (OF360) refers to Candida cvlindracea lipases obtained from Meito Sangyo Ltd., Tokyo, Japan.

The enantiomeric ratio (E) is calculated from Formula (13) above and the results are also shown in Table II. Also, in Table II:

$$c(\%) = \%\ \text{conversion} = \frac{ee_s}{ee_s + ee_p} \times 100$$

$ee_s$ = enantiomeric excess of the remaining substrate fraction (ester);

and

$ee_p$ = enantiomeric excess of the product fraction (alcohol).

**Table II**

| Ex. No | Lipase | Time (hrs) | Remaining substrate (22.1)(mg) | Product (22.3)(mg) | c (%) | $ee_s$ | $ee_p$ | E |
|---|---|---|---|---|---|---|---|---|
| 1 | P-30 | 23 | 37 | 37 | 51 | 99.5 | 97.6 | >100 |
| 2 | K-10 | 29 | 65 | 12 | 49 | 16.7 | 99.5 | >100 |
| 3 | AK | 89 | 54 | 20 | 35 | 53.5 | 99.0 | >100 |
| 4 | AP | 29 | 44 | 32 | 46 | 81.6 | 97 | >100 |
| 5 | PL | 98 | 64 | 9 | 8 | 9 | 99 | >100 |
| 6 | CCL | 288 | 48 | 7 | 8 | 9 | 99 | >100 |
| 7 | PPL | 240 | 68 | 2 | 5 | 5 | 97 | 77 |

Examples 8-12 Enzymatic resolution of (±)-cis-3-acetoxy-4-phenylazetidin-2-one.

A representative equation is as follows:

(23)

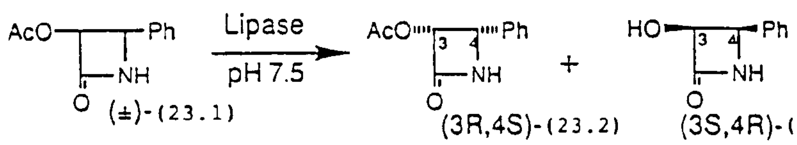


(±)-(23.1) (3R,4S)-(23.2) (3S,4R)-(23.3)

The racemate of Formula (23.1) is prepared as described by Holton on page 14 of EPA 0 400 971 A2.

A suspension consisting of 60 mg of the racemate of Formula (23.1) in 4 ml of 0.2 M aqueous phosphate

buffer (pH 6.8) was added either to 20 mg or to 40 mg of lipase (as indicated). The reaction mixture was stirred with a magnetic stirrer at 25°C until about 50% conversion was reached. The progress of the reaction was monitored by TLC.

The reaction mixture was extracted with ethyl acetate (3 x 25 ml), and the combined organic layer was dried over magnesium sulfate. After filtration, the organic solvent was evaporated in vacuo. The product and substrate were separated by silica gel flash chromatography using the solvent mixture ethyl acetate:hexane (2:1) and pure ethyl acetate as eluent. The $R_f$ value for Formula (23.2) was 0.5 and the $R_f$ valve for Formula (23.3) was 0.25 in the TLC system which consisted of ethyl acetate:hexane:ethanol (1:1:0.2).

The ee of the remaining substrate was determined by $^1H$ NMR ($CDCl_3$, 200 MHz) analysis of the acetyl group in the presence of the chiral shift reagent $Eu(hfc)_3$. The product of Formula (23.3) was first converted to the diacetyl derivative by reaction with acetic anhydride:pyridine (1:1) containing a trace of 4-dimethylaminopyridine, and the ee was then determined in a similar manner. The results of these enzymatic transformations are summarized in Table III.

**Table III**

| Ex. No. | Lipase | Time (hrs) | Remaining substrate (23.1)(mg) | Product (22.3) | c (%) | $ee_s$ | $ee_p$ | E |
|---|---|---|---|---|---|---|---|---|
| 8 | P-30 (40 mg) | 96 | 23 | 13 | 49 | 0.96 | 0.98 | 100 |
| 9 | AK (20 mg) | 69 | 30 | 19 | 67 | 0.67 | 1.00 | 100 |
| 10 | CCL (OF-360) (40 mg) | 31 | 40 | 15 | 26 | 0.35 | 0.97 | 50 |
| 11 | FAP (40 mg) | 78 | 31 | 15 | 40 | 0.62 | 0.94 | 52 |
| 12 | AP (20mg) | 16 | 14 | 40 | 66 | 1.00 | 0.66 | 12 |

Examples 13.1-13.7 Enzymatic enantioselective hydrolysis of cis-1-benzoyl-3-acetoxy-4-phenylazetidin-2-one.

A representative equation is as follows:

(24)
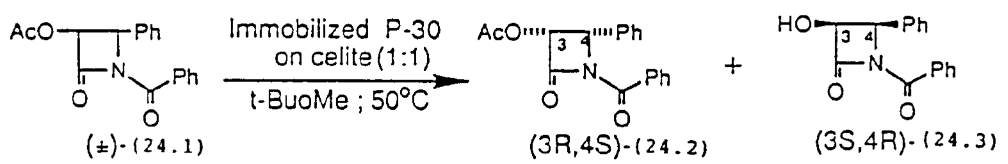

(±)-(24.1) (3R,4S)-(24.2) (3S,4R)-(24.3)

The racemate of Formula (24.1) was prepared from the racemate of Formula (23.1). A representative equation is as follows:

(24a)
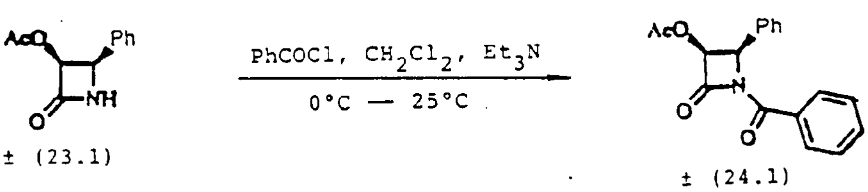

± (23.1) ± (24.1)

The preparation procedure was as follows:

To a solution of 2.5 g (0.01 mol) of racemic (±)-cis-3-acetoxy-4-phenylazetidin-2-one in 100 ml of dry methylene chloride was added a catalytic amount of dimethylaminopyridine and 17.4 ml (0.12 mol) of dry triethylamine. After the mixture was cooled to 0°C, 7.3 ml (0.06 mol) of benzoyl chloride was then added. The reaction mixture was stirred at 0°C and gradually warmed to 25°C under argon. After 16 hours, the reaction mixture was washed with water (3 x 100 ml). The organic layer was separated, dried over magnesium sulfate, and then evaporated to dryness in vacuo to give (±) (3.2 g) of the compound of Formula (24.1) as a white solid. Recrystallization of the crude product from ethyl acetate-hexane gave a compound having a melting point of 116-118°C. The $^1H$ 200 MHz NMR spectrum was consistent with the assigned structure.

Because the starting racemate of Formula (24.1) is unstable in aqueous medium, the biocatalytic hydrolytic reaction was conducted in anhydrous organic solvent with a small quantity of water. A series of experiments were carried out to establish the optimum conditions required for this transformation.

1) Effect of Temperature:

100 mg substrate and 200 mg of immobilized P-30 on Celite (1:1; Celite is a trade mark of Manville Products Corporation for its brand of diatomaceous earth) in 5 ml t-butyl methyl ether and 32 μL water (10 eq) was incubated at 50°C (and 40°C, 30°C, and 20°C, respectively) to about 50% conversion, monitored with TLC. The immobilized P-30 (prepared as described above) was separated from the reaction mixture by filtration, then washed with ethyl acetate. The combined organic solution was concentrated to dryness. The remaining substrate ($R_f$ = 0.66 in 1:1 ethylacetate:hexane) and product ($R_f$ = 0.40 in 1:1 ethylacetate:hexane) were separated by silica gel flash chromatography (5:1 hexane:ethyl acetate, 4:1 hexane:ethyl acetate as eluent).

Rotation of both the remaining substrate of Formula (24.1) and the products of Formulas (24.2) and (24.3) were checked in chloroform and ee was determined by $^1H$ NMR ($CDCl_3$, 200 MHz) analysis of the acetyl groups in the presence of the chiral shift reagent $Eu(hfc)_3$. The product of Formula (24.3) was first converted to starting material of Formula (24.1) by treatment with acetic anhydride, pyridine and 4-dimethylaminopyridine.

These results are tabulated in Table IV. In some cases, 80 mg of substrate of Formula (24.1) was used instead of 100 mg thereof. As a general rule, reaction rates increase about 2-3 fold for every 10°C increase in temperature. Since the enzyme appears to be fairly stable at 50°C, this is the most preferred temperature at which to conduct the incubation.

## Table IV
## Effect of Temperature

| Example | Reaction temperature (5, °C) | Reaction time (hrs) | Chemical yield | | | Rotation $[\alpha]_D$ (c=1.0, $CHCl_3$) | | | | Conversion (%) | E |
|---|---|---|---|---|---|---|---|---|---|---|---|
| | | | Remaining sub. (mg) | Product (mg) | Yield (%) | Remaining substrate | Product | $ee_S$ (%) | $ee_P$ (%) | | |
| 13.1 | 50 | 48 | 44.8 (100 mg)* | 37.4 | 42.5 | +137.3° (t=27°C) | -304.8° (t=27°C) | 97.5 | 99.5 | 49.5 | >100 |
| 13.2 | 40 | 62 | 35.9 (80 mg)* | 26.9 | 38.3 | +134.0° (t=29°C) | -284.0° (t=29°C) | 99.2 | 99 | 50.1 | >100 |
| 13.3 | 30 | 91.5 | 42.8 (80 mg)* | 24.4 | 34.7 | +92.5° (t=29°C) | -288.6° (t=29°C) | 69.7 | 99 | 41.3 | >100 |
| 13.4 | 20 | 94 | 66.8 (100 mg)* | 17.8 | 17.8 | +48.3° (t=29°C) | -278.0° (t=29°C) | 33.3 | 95 | 25.9 | 57 |

*Starting substrate.

2) Effect of Organic Solvent:

The following studies were conducted to determine which of the following organic solvents is the most suitable. The reaction mixture contained 80 mg substrate and 160 mg immobilized P-30 on Celite (1:1) in 4 ml organic solvent (t-butyl methyl ether, isopropyl ether, toluene, or a mixture of equal volume parts of each of dichloroethane, cyclohexane, and hexane) and 4.2 μL water (1.0 eq). The mixture was incubated at 50°C and the progress of the reaction was monitored by TLC. The isolation, purification and ee determinations were the same as described in relation to Table IV above.

The results are summarized in Table V.

The most suitable solvents are t-butyl methyl ether and isopropyl ether. Good reaction rates and high E values were obtained.

## Table V
## Effect of Organic Solvent

| Example | Solvent | Reaction time (hrs) | Chemical yield: Remaining sub. (mg) | Chemical yield: Product (mg) | Chemical yield: Yield (%) | Rotation $[\alpha]_D$ (c=1.0, $CHCl_3$): Remaining substrate | Rotation $[\alpha]_D$ (c=1.0, $CHCl_3$): Product | $ee_S$ (%) | $ee_P$ (%) | Conversion (%) | E |
|---|---|---|---|---|---|---|---|---|---|---|---|
| 13.5 | Ter-butyl methyl ether | 48 | 70.0* | 22.2 | 25.3 | +50.8° (t=27°C) | -276.5° (t=27°C) | 41.2 | 99 | 29.4 | >100 |
| 13.6 | Isopropyl ether | 49 | 51.8 | 29.0 | 27.0 | +57.6° (t=29°C) | -284.4° (t=29°C) | 44.4 | 99 | 30.9 | >100 |
| 13.7 | Toluene | 11 days | 56.3 | 9.8 | 13.9 | +20.2° (t=27°C) | -243.4° (t=27°C c=0.98) | 16.7 | 81.8 | 17.0 | 12 |
| 13.8 | Dichloromethane | 14 days | 55.4 | 9.5 | 10.8 | +182° (t=28°C) | -217.4° (t=28°C, c=0.98) | 4.8 | 93.3 | 4.8 | 43 |
| 13.9 | Cyclohexane | 15 days | 58.2 | 4.3 | 6.4 | +33.3° (t=26.5°C) | -280.2° (t=26.5°C, c=0.43) | 28.6 | 98.5 | 22.5 | >100 |
| 13.10 | Hexane | 16 days | 55.6 | 2.4 | 3.4 | +12.2° (t=28°C) | -183.3° (t=28°C, c=0.24) | 9.1 | 65.5 | 12.2 | 5 |

*100 mg of substrate was used.

3) Effect of Water:

To determine the optimum quantity of water that must be added to the organic solvent medium, the following studies were carried out.

Procedure:

100 mg substrate and 200 mg immobilized P-30 on Celite (1:1) in 5 ml organic solvent and varying quantities of water [(a) 52 μL, 10 eq; (b) 26 μL, 5 eq; and (c) 5.2 μL, 1 eq, respectively) were incubated at 50°C to about 50% conversion. The isolation and characterization procedures are the same as before. The results are shown in Table VI.

As much as 10 eq of water may be used although a small amount of non-enzymatic hydrolysis was observed. However, the reaction rate was faster than 5 eq of water. 10 eq of water is the preferred mode of operation.

Table VI
Effect of Water

| Example | Amount of water | Reaction time (hrs) | Chemical yield: Remaining sub. (mg) | Chemical yield: Product (mg) | Yield (%) | Rotation $[\alpha]_D$ (c=1.0, $CHCl_3$): Remaining substrate | Rotation: Product | $ee_s$ (%) | $ee_p$ (%) | Conversion (%) | E |
|---|---|---|---|---|---|---|---|---|---|---|---|
| 13.11 | 10 eq | 48 | 44.8 | 37.4 | 42.5 | +137.3° (t=27°C) | -304.8° (t=27°C) | 97.5 | 99.5 | 49.5 | >100 |
| 13.12 | 5 eq | 67.5 | 34.9* | 24.7 | 33.1 | +130.0° (t=29°C) | -231.5° (t=29°C) | 95.9 | 99 | 49.2 | >100 |
| 13.13 | 1 eq | 187 | 50.9 | 25.9 | 29.5 | +70.2° (t=26°C) | -257.0° (t=26°C) | 55.6 | 99 | 36.0 | >100 |

*80 mg of substrate was used instead of 100 mg.

4) Recycling of the Immobilized P-30 Lipase:

The P-30 lipase when immobilized on Celite is very stable in the organic solvents described herein in the presence of a small quantity of water at 50°C.

Procedure:

The procedure is the same as before. After filtration, the immobilized P-30 was reused, and the same process was repeated several times. 200 mg Celite/P-30 and 100 mg substrate were also used. However, in the third cycle, the amount of Celite/P-30 was reduced to 190 mg, and substrate to 95 mg. In the fourth cycle, Celite/P-30 was 180 mg and substrate was 90 mg. In each case, the amount of t-methyl ether and water was reduced accordingly. The results are shown in Table VII below. Table VII shows that the same batch of lipase may be used for at least 4 cycles without loss of enantioselectivity.

This result demonstrates the economy of the process because the cost of the lipase may be reduced at least four-fold or more.

## Table VII
## Recycling of the Immobilized P-30 Lipase

| Example | Celite P30 cycle | Reaction time (hrs) | Chemical yield: Remaining sub. (mg) | Chemical yield: Product (mg) | Yield (%) | Rotation $[\alpha]_D$ (c=1.0, $CHCl_3$): Remaining substrate | Rotation: Product | $ee_S$ (%) | $ee_P$ (%) | Conversion (%) | E |
|---|---|---|---|---|---|---|---|---|---|---|---|
| 13.14 | 1 | 48 | 44.8 | 37.4 | 42.5 | +137.3° (t=27°C) | -304.8° (t=27°C) | 97.5 | 99.5 | 49.5 | >100 |
| 13.15 | 2 | 66 | 43.3 | 44.8 | 51.0 | +134.6° (t=29.5°C) | -286.2° (t=29.5°C) | 97.5 | 99 | 49.5 | >100 |
| 13.16 | 3 | 121.5 | 44.1* | 30.5 | 36.5 | +112.4° (t=29.5°C) | -284.5° (t=29.5°C) | 86.1 | 99 | 46.5 | >100 |
| 13.17 | 4 | 144 | 42.2† | 35.0 | 44.3 | +132.1° (t=26.5°C) | -270.0° (t=26.5°C) | 94.3 | 99 | 43.3 | >100 |

*95 mg of substrate.

†90 mg of substrate.

Examples 14-16. Enantioselective transesterification of (±)-cis-1-p-methoxyphenyl-3-hydroxy-4-phenylazetidin-2-one (±-25.1).

A representative equation is as follows:

(25)

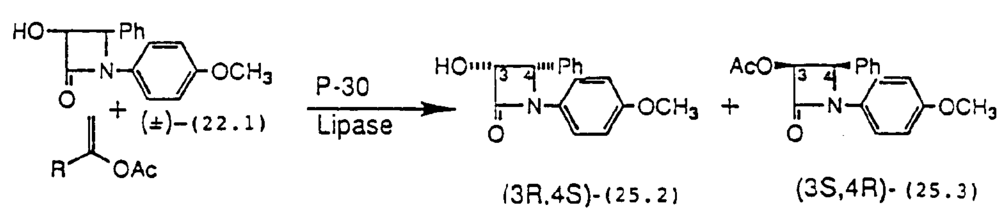


(±)-(22.1)

(3R,4S)-(25.2) (3S,4R)-(25.3)

The racemate of Formula (25.1) is prepared as described by Holton on page 14 of EPA 0 400 971 A2.

The separation of the antipodes of product of Formula (25.2) may also be achieved by means of enantioselective transesterification with the P-30 lipase. In this case, the (3S,4R)-enantiomer is preferentially converted into its acetoxy derivative.

Procedure:

The reaction mixture contained: 40 mg of (±) 25.1; 20 mg of P-30 lipase, either 3 ml of isopropenyl acetate or vinyl acetate as indicated, with or without t-butyl-methyl ether as solvent. The other procedures are the same as those described in Example I.

The results are summarized in Table VIII.

**Table VIII**

| Enantioselective Transesterification of Substrate of Formula (25.1) | | | | | | | |
|---|---|---|---|---|---|---|---|
| Ex. No. | R | Solvent | Time | $ee_S$ (25.1) | $ee_P$ (25.3) | c(%) | E |
| 14 | $CH_3$ | None | 4 days | 0.44 | 1 | 33 | >100 |
| 15 | $CH_3$ | But-O-Me | 4 days | 0.2 | 1 | 17 | 99 |
| 16 | H | But-O-Me | 4 days | 0.87 | 1 | 47 | >100 |

Examples 17.1-17.4 Enantioselective transesterification of (±)-cis-3-hydroxy-4-phenylazetidin-2-one.

A representative equation is as follows:

(26)

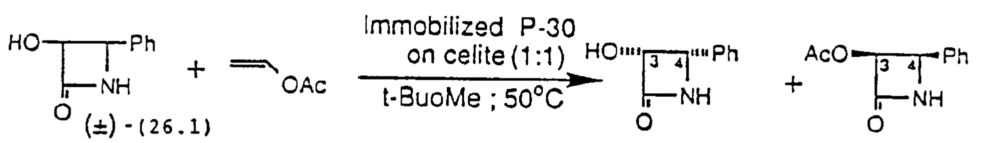


(±)-(26.1)

(3R,4S)-(26.2) (3S,4R)-(26.3)

The racemate of Formula (26.1) is prepared as described by Holton on page 14 of EPA 0 400 971 A2.

Procedure:

The reaction mixture contained 33 mg of the racemate 26.1, 3 equivalents of vinyl acetate, 40 mg of P-30 lipase immobilized on Celite (1:1) in 3 ml of t-butyl-o-methyl ether. The workup procedure, the isolation and ee determination are the same as those described in Example 1. The results are summarized in Table IX.

### Table IX
### Enantioselective Transesterification of Substrate of Formula (26.1)

| Example | Cycles | Reaction time (hrs) | Temperature | Substrate (26.1) | | | Product (26.3) | | | Conversion (%) | E |
|---|---|---|---|---|---|---|---|---|---|---|---|
| | | | | Yield (%) | $[\alpha]_D^{21°}$ (MeOH) | $ee_S$ | Yield (%) | $[\alpha]_D^{21°}$ ($CHCl_3$) | $ee_P$ | | |
| 17.1 | 1 | 54 | 50°C | 52.2 | +115.9° (c=0.17) | 0.63 | 29.3 | +50.8° (c=0.39) | 1 | 0.38 | >100 |
| 17.2 | 2 | 76 | 50°C | 52.2 | +109.2° (c=0.26) | 0.66 | 32 | +45.4° (c=0.35) | 1 | 40 | >100 |
| 17.3 | 3 | 72 | 50°C | 47.5 | +90.3° (c=0.29) | 0.59 | 27.5 | +49.7° (c=0.32) | 0.95 | 38 | 96 |
| 17.4 | 4 | 77 | 50°C | 48.5 | +69.4° (c=0.22) | 0.41 | 22 | +48.5° (c=0.22) | 0.98 | 30 | >100 |

Preparation of Taxol-Type Compounds

Example 18: Taxol Preparation

a) cis-1-benzoyl-3R-(1-ethoxyethoxy)-4S-phenylazetidin-2-one (28)

(27) 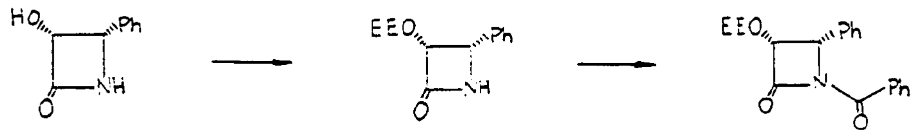

(26.2) (27.1) (27.2)

To a solution of 340 mg (21 mmol) of cis-3R-hydroxy-4S-phenylazetidin-2-one (Formula 26.2) in 2 ml of THF at 0°C was added 0.5 ml of ethyl vinyl ether and 2 mg of methane sulfuric acid. The mixture was stirred at 0°C for 20 minutes, diluted with 2 ml of saturated aqueous sodium bicarbonate and then extracted with three 5 ml portion of ethyl acetate. The combined ethyl acetate layers were dried over magnesium sulfate and concentrated to give 420 mg of cis-3R-(1-ethoxyethoxy)-4S-phenylazetidin-2-one (27) as an oil.

To a solution of 235 mg (1 mmol) of cis-3R-(1-ethoxyethoxy)-4S-phenylazetidin-2-one (27) in 5 ml of THF at -78°C was added 0.4 ml (1 mmol) of a 2.5 M solution of n-butyllithium in hexane. The mixture was stirred for 20 minutes at -78°C and a solution of 140 mg (1 mmol) of benzoyl chloride in 1 ml of THF was added. The mixture was stirred at -78°C for 60 minutes and diluted with 10 ml of saturated aqueous sodium bicarbonate and then extracted with three 10 ml portions of ethyl acetate. The combined ethyl acetate extract was dried over magnesium sulfate and concentrated to yield an oily residue (300 mg). Silica gel column chromatography (ethyl acetate-hexane) afforded 250 mg of cis-1-benzoyl-3R-(1-ethoxyethoxy)-4S-phenylazetidin-2-one (28). (The procedure is as described by Holton on pages 14 and 15 of EPA 0 400 971 A2 for the preparation of the racemate).

b) Preparation of Taxol

0.32 mmol of cis-1-benzoyl-3R-(1-ethoxyethoxy)-4S-phenylazetidin-2-one (the compound of Formula 28) is added to 0.064 mmol of 7-O-triethylsilyl baccatin III (see Formula (5) prepared as described by Greene et al.), 0.064 mmol of 4-dimethylaminopyridine and 0.032 ml of pyridine in a small reaction vessel (as described by Holton at page 15 of EPA 0 400 971 A2). The mixture is stirred for 12 hours and diluted with 100 ml of ethyl acetate. The ethyl acetate solution is extracted with 20 ml of 10% aqueous copper sulfate solution, dried over sodium sulfate and concentrated under vacuum. The residue is filtered through a plug of silica gel eluted with ethyl acetate. Flash chromatography on silica gel eluted with ethyl acetate/hexane followed by recrystallization from ethyl acetate/hexane gives a C-13 ester of 7-O-triethylsilyl baccatin III.

A sample of the ester is dissolved in 2 ml of ethanol and 0.5 ml of 0.5% aqueous HCl is added. The mixture is stirred at 0°C for 30 hours and is diluted with 50 ml ethyl acetate. The solution is extracted with 20 ml of saturated aqueous sodium bicarbonate solution, dried over sodium sulfate and concentrated. The residue is purified by column chromatography on silica gel eluted with ethyl acetate/hexane to provide a product which is identical with taxol.

Thus, the β-lactam intermediate of Formula (28) is directly coupled to baccatin III-type compounds to form taxol.

Examples 19-22 Preparation of Non-Natural Taxol-Type Compounds

(28) 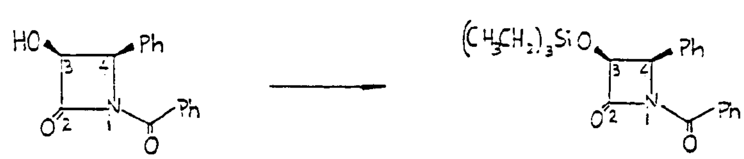

(24.3) (29)

a) cis-1-benzoyl-3S-triethylsiloxy-4R-phenylazetidin-2-one

To a stirred solution of 1 mmol of cis-1-benzoyl-3S-hydroxy-4R-phenylazetidin-2-one (Formula 24.3) in 10 ml of dichloromethane (cooled to 0°C) was added 2 mmol of triethylamine. To this mixture was added 1.1 mmol of triethyltrifluoromethane sulfate and the reaction mixture was stirred for 30 minutes at 0°C. The contents were poured into 10 ml of water and the mixture was extracted with diethyl ether (30 ml x 2). The combined organic layer was washed with 5 ml of sodium chloride solution, and dried over magnesium sulfate. After filtration, the organic solvent was concentrated to dryness under reduced pressure to yield the triethylsilyl derivative (29) in quantitative yield.

b) Following the procedure of Example 18, various starting alcohols of Formula (4) are coupled to the compound of Formula (29) to produce taxol-type compounds of generic Formula (20). The resulting compounds are shown below in Table X.

Table X

Exemplary Taxol-Type Compounds

| Ex. No. | Formula No. of Starting Alcohol I.D. No. (from Table I) | Formula No. of Starting β-Lactam Enantiomer | Taxol-Type Product Compound |
|---|---|---|---|
| 19 | 4.1 | 29 | 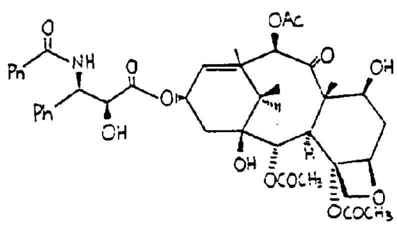 |
| 20 | 4.6 | 29 | 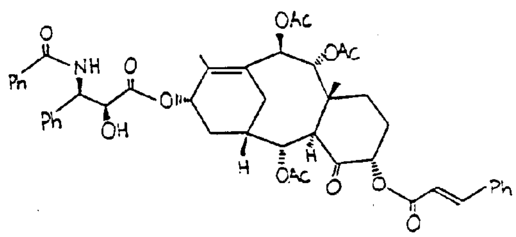 |
| 21 | 4.3 | 29 | 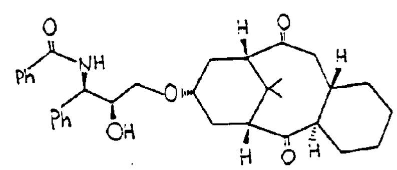 |
| 22 | 4.4 | 29 | 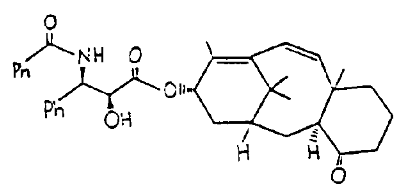 |

Examples 23-26:Preparation of Natural Type Taxol-Type Compounds

Following the procedure of Example 18, various starting alcohols of generic Formula (4) are coupled to the starting β-lactam compounds of Formula (27.2) to produce particular taxol-type compounds of generic Formula (19) as shown below in Table XI.

Table XI

Exemplary Taxol-Type Compounds

| Ex. No. | Formula No. of Starting Alcohol I.D. No. (from Table I) | Formula No. of Starting $\beta$-Lactam Enantiomer | Taxol-Type Product Compound |
|---|---|---|---|
| 23 | 4.1 | 28 | 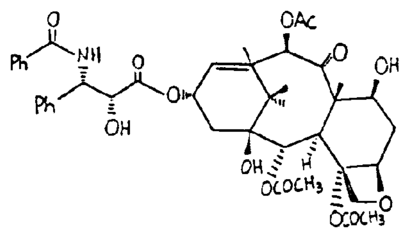 |
| 24 | 4.6 | 28 | 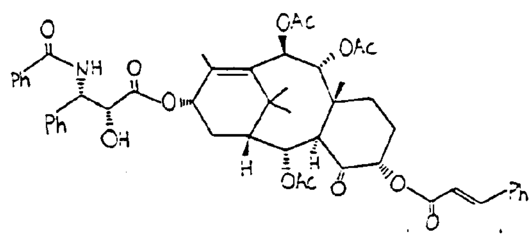 |
| 25 | 4.3 | 28 | 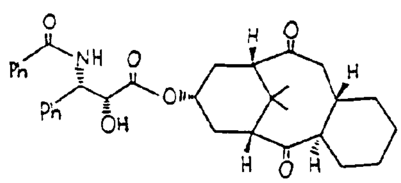 |

Table XI (Continued)

| | | | |
|---|---|---|---|
| 26 | 4.4 | 28 | 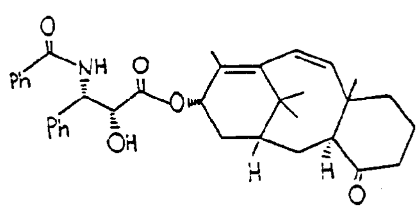 |

## Claims

1. A process for preparing a taxol-type compound comprising the steps of:
(a) contacting a lipase under liquid phase conditions with a starting β-lactam of the formula:

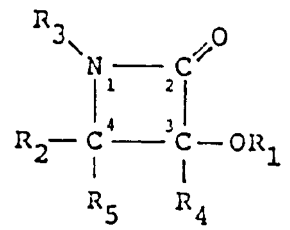

wherein:
$R_1$ is a radical selected from the group consisting of hydrogen, lower alkanoyl, lower α-haloalkanoyl and lower alkenoyl,
$R_2$ is a radical selected from the group consisting of hydrogen, aryl, substituted aryl, aralkyl, lower alkyl and lower alkenyl,
$R_3$ is a radical selected from the group consisting of hydrogen, hydroxyl, lower alkanoyl, lower alkenoyl and aryloyl,
$R_4$ is a radical selected from the group consisting of hydrogen, lower alkyl and lower alkenyl,
$R_5$ is a radical selected from the group consisting of hydrogen, lower alkyl and lower alkenyl; and
(b) separating from the resulting reaction medium a β-lactam enantiomer which is either (3R,4S) or (3S,4R) and which has one of the following generic formulas:

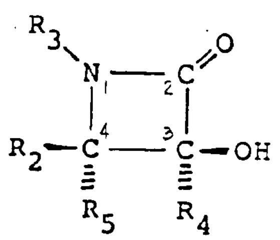

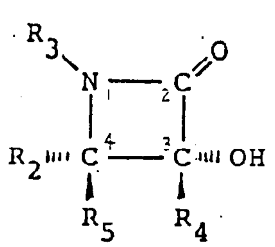

where $R_2$, $R_3$, $R_4$ and $R_5$ are as defined above.

2. The process of claim 1 wherein said β-lactam enantiomer is coupled to the C-13 hydroxyl group of a starting alcohol under liquid phase conditions in the presence of a sufficient amount of an activating agent, thereby to form an ester moiety at said C-13 position and produce a taxol-type compound, said starting alcohol having the generic structure:

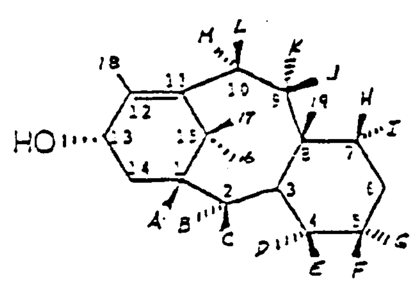

wherein:
A is a radical selected from the group consisting of hydrogen, hydroxyl, triethylsilyloxy, lower alkyl, lower alkenyl, lower alkoxy, lower alkanoyloxy, lower alkenoyloxy and aryloyloxy,
B and C are each an independently selected radical from the group consisting of hydrogen, hydroxyl, triethylsilyloxy, lower alkanoyloxy, lower alkenoyloxy, aryloyloxy and cyclohexoyloxy,
B and C taken together can form an oxo radical,

D is a radical selected from the group consisting of hydrogen, hydroxyl, lower alkanoyloxy, lower alkenoyloxy and aryloyloxy,

D and E taken together can form a radical selected from the group consisting of oxo and methylene,

E and F together with the carbon atoms between them can form an oxetane (i.e., an oxycyclobutyl) ring,

F and G are each an independently selected radical from the group consisting of hydrogen, hydroxyl, triethylsilyloxy, lower alkanoyloxy, lower alkenoyloxy, aryloyloxy and phen(lower alkan)oyloxy,

F and G taken together can form an oxo radical,

H and I are each an independently selected radical from the group consisting of hydrogen, hydroxyl, triethylsilyloxy, lower alkanoyloxy, lower alkenoxyloxy and aryloyloxy,

J and K are each an independently selected radical from the group consisting of hydrogen, hydroxyl, triethylsilyloxy, lower alkanoyloxy, lower alkenoyloxy and aryloyloxy,

J and K taken together can form an oxo radical,

L and M are each an independently selected radical from the group consisting of hydrogen, hydroxyl, triethylsilyloxy, lower alkanoyloxy, lower alkenoyloxy and aryloyloxy, and

L and M together can form an oxo radical.

**3.** The process of claim 2 wherein said resulting taxol-type compound is purified.

**4.** The process of claim 2 wherein said starting alcohol includes at least one protective tri (lower alkyl) silyl group, and wherein, after said coupling, said resulting taxol-type compound is subjected to mild hydrolyzing conditions sufficient to remove said tri (lower alkyl) silyl group(s).

**5.** The process of claim 4 wherein said resulting hydrolyzed product is purified.

**6.** The process of claim 1 wherein said liquid phase is aqueous and said starting β-lactam and said lipase are each dissolved therein.

**7.** The process of claim 1 wherein said liquid medium is a non-lipase denaturing organic liquid in which said starting β-lactam and said lipase are each dissolved, and wherein a starting 1-(substituted) ethenyl acetate of the formula

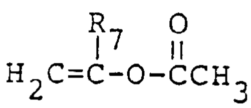

wherein $R_7$ is a radical selected from the group consisting of hydrogen and lower alkyl is additionally dissolved.

**8.** The process of claim 3 wherein the mole ratio of said starting β-lactam to said starting acetate is in the range of about 1 to about 3.

**9.** The process of claim 1 wherein said contacting is carried out at a temperature in the range of about 10°C to about 50°C.

**10.** The process of claim 1 wherein said contacting is carried out in said liquid phase at a pH that is in the range of about 3 to about 10.

**11.** The process of claim 1 wherein said starting β-lactam has a concentration in said liquid phase that is in the range of about 0.1 to about 1 M.

**12.** The process of claim 1 wherein said liquid phase is aqueous and is phosphate buffered.

**13.** The process of claim 1 wherein the weight ratio of said starting β-lactam to said lipase is in the range of about 1:1 in said liquid medium.

**14.** The process of claim 1 wherein said lipase is a member of the Pseudomonas genus.

**15.** The process of claim 14 wherein said starting β-lactam is hydrolyzed during said contacting under aqueous

phase conditions in the presence of at least one lipase that is selected from the group consisting of P-30, K-10, AK, AP, PL, CCL, PPL and FAP, and wherein, in said starting β-lactam, $R_1$ is selected from the group consisting of lower alkanoyl and lower alkenoyl.

**16.** The process of claim 14 wherein said starting β-lactam is transesterified with a starting 1-(substituted) ethenyl acetate of the formula

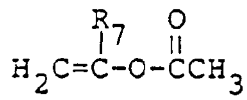

where $R_7$ is a radical selected from the group consisting of hydrogen and lower alkyl under organic phase conditions in the presence of at least one lipase that is selected from the group consisting of P-30 and AK.

**17.** The process of claim 1 wherein said contacting is carried out until at least about 50 weight percent of said starting β-lactam has been converted into a mixture of enantiospecific β-lactam products.

**18.** The process of claim 1 wherein said separating is carried out by the steps comprising:
(a) removing said lipase from the liquid medium resulting from said contacting; and
(b) subjecting the resulting liquid medium to chromatography with a non-miscible organic liquid eluent to separate said enantiospecific β-lactam.

**19.** The method of claim 1 wherein said liquid medium is aqueous and contains a phosphate buffer, and wherein, during said contacting, enantioselective hydrolysis is accomplished, such that one class of product enantiomers is produced wherein the members of such class have the (3S,4R) configuration.

**20.** The method of claim 1 wherein said liquid medium is organic and further includes a starting ester having the formula

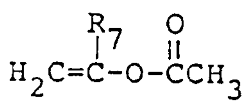

where $R_7$ is selected from the group consisting of hydrogen and lower alkyl, and wherein, during said contacting, enantioselective transesterification is accomplished, such that one class of product enantiomers is produced wherein $R_1$ is hydroxyl and wherein the members of said class have the (3R,4S) configuration.

**21.** The method of claim 1 wherein said lipase is characterized by the capacity to produce during said contacting an enantiomeric excess of said β-lactam enantiomer product which is at least about 100 times greater than the amount of other β-lactam compounds concurrently produced.

**22.** The method of claim 1 wherein said lipase is recycled.

**23.** 3-Hydroxy substituted β-lactam enantiomers having the (3S,4R) structure and characterized by the formula:

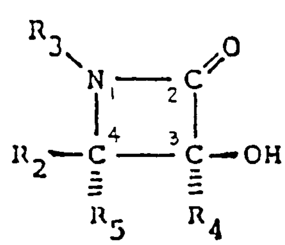

where

$R_2$ is a radical selected from the group consisting of hydrogen, aryl, substituted aryl, aralkyl, lower alkyl and lower alkenyl,

$R_3$ is a radical selected from the group consisting of hydrogen, hydroxyl, lower alkanoyl, lower alkenoyl and aryloyl,

$R_4$ and $R_5$ are each independently selected from the group consisting of hydrogen, lower alkyl and lower alkenyl.

**24.** 3-Hydroxy substituted β-lactam enantiomers having the (3R,4S) structure and characterized by the formula:

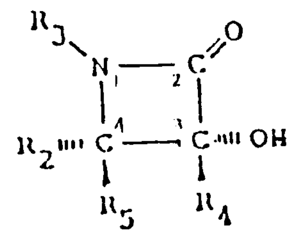

where

$R_2$ is a radical selected from the group consisting of hydrogen, aryl, substituted aryl, aralkyl, lower alkyl and lower alkenyl,

$R_3$ is a radical selected from the group consisting of hydrogen, hydroxyl, lower alkanoyl, lower alkenoyl and aryloyl, and

$R_4$ and $R_5$ are each independently selected from the group consisting of lower alkyl and lower alkenyl.

**25.** Taxol-type compounds of the formula:

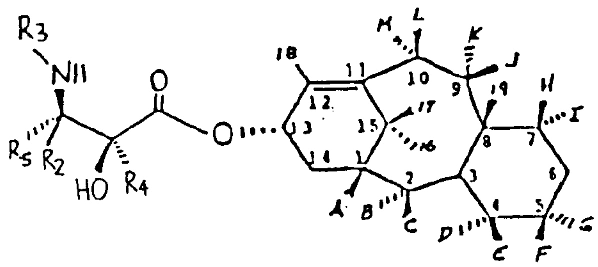

wherein:

$R_2$ is a radical selected from the group consisting of hydrogen, aryl, substituted aryl, aralkyl, lower alkyl and lower alkenyl,

$R_3$ is a radical selected from the group consisting of hydrogen, hydroxyl, lower alkanoyl, lower alkenoyl and aryloyl,

$R_4$ and $R_5$ are each independently selected from the group consisting of hydrogen, lower alkyl and lower alkenyl,

A is a radical selected from the group consisting of hydrogen, hydroxyl, triethylsilyloxy, lower alkyl, lower alkenyl, lower alkoxy, lower alkanoyloxy, lower alkenoyloxy and aryloyloxy,

B and C are each an independently selected radical from the group consisting of hydrogen, hydroxyl, triethylsilyloxy, lower alkanoyloxy, lower alkenoyloxy, aryloyloxy and cyclohexoyloxy,

B and C taken together can form an oxo radical,

D is a radical selected from the group consisting of hydrogen, hydroxyl, lower alkanoyloxy, lower alkenoyloxy and aryloyloxy,

D and E taken together can form a radical selected from the group consisting of oxo and methylene,

E and F together with the carbon atoms between them can form an oxetane (i.e., an oxycyclobutyl) ring,

F and G are each an independently selected radical from the group consisting of hydrogen, hydroxyl, triethylsilyloxy, lower alkanoyloxy, lower alkenoyloxy, aryloyloxy and phen(lower alkan)oyloxy,

F and G taken together can form an oxo radical,

H and I are each an independently selected radical from the group consisting of hydrogen, hydroxyl, triethylsilyloxy, lower alkanoyloxy, lower alkenoxyloxy and aryloyloxy,

J and K are each an independently selected radical from the group consisting of hydrogen, hydroxyl, triethylsilyloxy, lower alkanoyloxy, lower alkenoyloxy and aryloyloxy,

J and K taken together can form an oxo radical,

L and M are each an independently selected radical from the group consisting of hydrogen, hydroxyl, triethylsilyloxy, lower alkanoyloxy, lower alkenoyloxy and aryloyloxy, and

L and M together can form an oxo radical.

**26.** Taxol-type compounds of the formula:

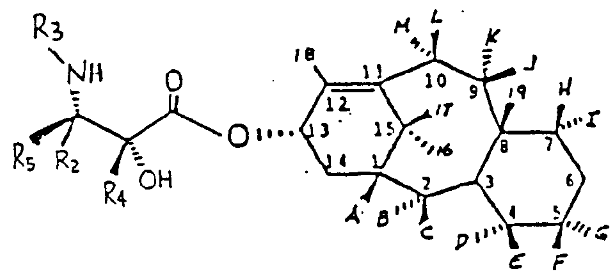

wherein:

$R_2$ is a radical selected from the group consisting of hydrogen, aryl, substituted aryl, aralkyl, lower alkyl and lower alkenyl,

$R_3$ is a radical selected from the group consisting of hydrogen, hydroxyl, lower alkanoyl, lower alkenoyl and aryloyl,

$R_4$ and $R_5$ are each independently selected from the group consisting of lower alkyl and lower alkenyl,

A is a radical selected from the group consisting of hydrogen, hydroxyl, triethylsilyloxy, lower alkyl, lower alkenyl, lower alkoxy, lower alkanoyloxy, lower alkenoyloxy and aryloyloxy,

B and C are each an independently selected radical from the group consisting of hydrogen, hydroxyl, triethylsilyloxy, lower alkanoyloxy, lower alkenoyloxy, aryloyloxy and cyclohexoyloxy,

B and C taken together can form an oxo radical,

D is a radical selected from the group consisting of hydrogen, hydroxyl, lower alkanoyloxy, lower alkenoyloxy and aryloyloxy,

D and E taken together can form a radical selected from the group consisting of oxo and methylene,

E and F together with the carbon atoms between them can form an oxetane (i.e., an oxycyclobutyl) ring,

F and G are each an independently selected radical from the group consisting of hydrogen, hydroxyl, triethylsilyloxy, lower alkanoyloxy, lower alkenoyloxy, aryloyloxy and phen(lower alkan)oyloxy,

F and G taken together can form an oxo radical,

H and I are each an independently selected radical from the group consisting of hydrogen, hydroxyl, triethylsilyloxy, lower alkanoyloxy, lower alkenoxyloxy and aryloyloxy,

J and K are each an independently selected radical from the group consisting of hydrogen, hydroxyl, triethylsilyloxy, lower alkanoyloxy, lower alkenoyloxy and aryloyloxy,

J and K taken together can form an oxo radical,

L and M are each an independently selected radical from the group consisting of hydrogen, hydroxyl, triethylsilyloxy, lower alkanoyloxy, lower alkenoyloxy and aryloyloxy, and

L and M together can form an oxo radical.